# EUROPEAN PATENT SPECIFICATION

(11) **EP 3 470 045 B1**
(45) Date of publication and mention of the grant of the patent: **02.09.2020**
(21) Application number: 18199563.0
(22) Date of filing: 10.10.2018
(51) Int. Cl.: A61J 1/10, B32B 27/08, B32B 27/30, B32B 27/32

(54) **INTRAVENOUS INFUSION DOSAGE FORM FOR PEMETREXED**
INTRAVENÖSE INFUSIONSDARREICHUNGSFORM FÜR PEMETREXED
FORME POSOLOGIQUE DE PERFUSION INTRAVEINEUSE DE PEMETREXED

(30) Priority: 10.10.2017 IN 201721035954
(43) Date of publication of application: 17.04.2019
(73) Proprietor: Sun Pharmaceutical Industries Ltd, 400063 Mumbai Maharashtra (IN)
(72) Inventor: KUMAR, Samarth, 390020 Gujarat (IN); VARU, Ramaji Karshanbhai, 390020 Gujarat (IN); PATEL, Nishit, 390020 Gujarat (IN); KANE, Prashant, 390020 Gujarat (IN); BHOWMICK, Subhas Balaram, 390020 Gujarat (IN)
(74) Representative: Harrison IP Limited

(56) References cited:
- WO-A1-2016/129000
- US-A1- 2012 215 196
- US-A1- 2017 197 769

## Description

### FIELD OF THE INVENTION

The present invention relates to an intravenous infusion dosage form of pemetrexed and a process for its preparation.

### BACKGROUND OF THE INVENTION

Pemetrexed is used in the treatment of malignant pleural mesothelioma and non-small cell lung cancer. It is known (see Jansen PJ et al, Journal of Pharmaceutical Sciences, Volume 105, Issue 11, November 2016, Pages, 3256-3268) that pemetrexed undergoes degradation via different mechanisms, like oxidation, hydrolysis, dimerization and others that are unknown and not yet elucidated. In view of the instability, the first commercial product, ALIMTA®, was supplied as a sterile lyophilized powder for intravenous infusion and made available in single-dose vials and is marketed in the United States by Eli Lilly. ALIMTA® requires reconstitution in the hospital setting and is thus not a ready to infuse intravenous infusion dosage form.

US 6,686,365 (the '365 patent) assigned to Eli Lilly noted the desirability of avoiding a freeze-drying technique and disclosed a pre-concentrate comprising pemetrexed; at least one antioxidant selected from a group consisting of monothioglycerol, L-cysteine, and thioglycolic acid; and a pharmaceutically acceptable excipient. The pre-concentrate required dilution in a hospital setting and is thus not a large volume ready to infuse intravenous infusion dosage form.

WO 2013/179,310 (the '310 patent application) assigned to Mylan describes a concentrated aqueous parenteral composition of pemetrexed disodium comprising at least one stability enhancing adjuvant such as a cyclodextrin derivative. The concentrated aqueous parenteral composition required dilution before administration and is thus not a large volume ready to infuse intravenous infusion dosage form.

US 2013/0231,357 (the '357 patent application) assigned to Eagle Pharmaceuticals discloses pemetrexed containing liquid pharmaceutical compositions to be diluted before administration, the compositions comprising antioxidants selected form lipoic acid, dihydrolipoic acid, methionine and mixtures thereof; a chelating agent selected from lactobionic acid, sodium citrate, tribasic or mixtures thereof. A large volume ready to infuse solution, however, is not disclosed.

WO 2013/179,248 (the '248 patent application) assigned to Fresenius Kabi Oncology Ltd. relates to a pharmaceutical composition of pemetrexed which is either a liquid ready to use solution formulation or a lyophilized pharmaceutical composition for parenteral administration comprising a pharmaceutically acceptable organic amine as a stabilizer, an inert gas and can have antioxidants, chelating agents, amino acids, preservatives etc. The liquid ready to use solution formulation disclosed in '248 application required dilution before administration and did not disclose a large volume ready-to-infuse intravenous infusion dosage form.

CN Patent No. 101081301, assigned to Hainan Tianyuankangze Pharmaceutical Technology Co. Ltd. again discloses a ready to dilute formulation of pemetrexed stabilized by using antioxidant like L-arginine, L-glutathione, L- methionine and L-tryptophan. It is not a large volume ready-to-infuse intravenous infusion dosage form.

WO 2013/144,814 (the '814 patent application) assigned to Fresenius Kabi Oncology Ltd. describes a concentrated solution of pemetrexed to be diluted before administration, which avoids the use of excipients such as anti-oxidants, chelating agents, complexing agents such as cyclodextrins and amino acids. The concentrated pemetrexed solution disclosed therein was stabilized by strict control of oxygen content of drug solution and vial headspace with the use of an inert gas. No large volume ready-to-infuse intravenous infusion dosage form, however, is disclosed.

WO2012/121,523 (the '523 application) assigned to Kuhnil Pharm describes a method for preparing a pharmaceutical formulation in the form of an antioxidant-free solution for injection, the method of which comprises: (a) controlling a dissolved oxygen concentration in a solution for injection comprising pemetrexed or its salt by various degassing methods such as purging of the aqueous vehicle or solution and (b) filling a container for injection with the solution obtained from the step (a), in a glove box. The '523 application discusses conventional closed systems such as glove bag to control oxygen during filling operation. However, such processes which makes use of closed systems like globe box during filling involve manual handling operation and is not feasible for large-scale commercial manufacturing of a parenteral product under GMP environment in a pharmaceutical plant. The '523 application discloses concentrated solution of pemetrexed in a vial to be diluted before use and thus is not a large volume ready-to-infuse intravenous infusion dosage form.

There exists a need for a large volume, ready-to-infuse intravenous infusion dosage form of pemetrexed. Particularly, there is a need for ready-to-infuse intravenous infusion dosage form that is also autoclavable. Applicant's own patent publication WO2016/129000 (hereinafter referred to as '9000 application) in fact was the first disclosure of a ready to infuse infusion dosage form of pemetrexed. The '9000 application discloses a method for preparing the intravenous infusion dosage form comprising steps of-
a. dissolving pemetrexed or its pharmaceutically acceptable salt and an osmogent in a parenteral aqueous vehicle,
b. filling the solution of step (a) into a flexible infusion container,
c. sealing the filled flexible infusion container,
d. surrounding the flexible infusion container by a secondary packaging/container and sealing the secondary packaging/container,
e. subjecting the container of step (d) to moist heat sterilization,
wherein in each of the above steps, low oxygen conditions are maintained in the solution and/or in the head space of the flexible infusion container and in the space between the flexible primary infusion container and secondary packaging/container. The publication disclosed a ready-to-infuse intravenous infusion dosage form with large volume of an aqueous solution of pemetrexed from 50 ml to 1000 ml. One disadvantage of the method was that it required that the aqueous solution in the first container of the infusion dosage form be visually inspected after step e. To enable visual inspection of the contents of the first container, it is necessary to remove the first container from the secondary packaging/container. Further, after visual inspection it is necessary to again place the first container in a secondary packaging/container and restore the inert atmosphere in the space between the two containers. These requirements make the process cumbersome. There remains the need to provide a large volume, ready-to-infuse intravenous infusion dosage form of pemetrexed that has prolonged stability when stored at room temperature and that can be autoclaved.

### OBJECTS OF THE PRESENT INVENTION

It is an object of the present invention to manufacture a stable, autoclavable, intravenous infusion dosage form by a simple process. Particularly, it was an object to avoid the disadvantages associated with the process of the '9000 application, namely autoclaving the first container without the need to surround the first container with a secondary packaging and without the need to place an oxygen scavenger or fill an inert gas in the space between the first primary container and the secondary packaging.

It is also an object of the present invention to prepare an intravenous infusion dosage form of pemetrexed that is autoclavable (i.e. which can withstand the harsh conditions of autoclaving), and still have prolonged stability at room temperature as terminal sterilization by autoclaving provides highest assurance of sterility for parenteral dosage forms.

It is another object of the present invention to provide an intravenous infusion dosage form with a large volume of an aqueous solution of pemetrexed that uses minimum amount of excipients. It is an object of the present invention to avoid the use of antioxidants, complexing agents like cyclodextrins, chelating agents and amino acids in the large volume aqueous solution of pemetrexed.

Prior to the present invention, inventors had faced a problem in that infusion flexible plastic containers not free of a polyamide when employed for intravenous infusion dosage form for pemetrexed were subjected to autoclaving for terminal sterilization, particles of unknown nature were found to be generated. The chemical nature of these particles was investigated by separating the particles from the aqueous solution that were generated during autoclaving. The filtered particles were then subjected to structural characterization techniques such as Raman spectroscopy and Mass Spectroscopy (LC-MS/MS). It was found that these particles were of polyamide-11 cyclic dimer and/or polyamide-11 cyclic trimer (hereinafter referred to as "polyamide particles"). It is believed that these particles may have been originated from one of the layers of the plastic container made up of polyamide. Without wishing to be bound by any theory, the inventors believed that upon autoclaving, the polyamide-11 cyclic dimer and/or polyamide-11 cyclic trimer may have migrated from the polyamide layer into the aqueous solution of pemetrexed. Such polyamide particles may have toxicological and/or regulatory implications and are hazardous to health.

The present invention solved this problem. The present invention provided an intravenous infusion dosage form of pemetrexed in a multilayered flexible plastic container that can be directly administered to the patients intravenously, and which allows autoclaving the first container without the need to surround the first container with a secondary packaging. While attempting to arrive at such intravenous infusion dosage form of pemetrexed in a multilayered flexible plastic container the present inventors discovered that the multilayer flexible plastic container needed to be necessarily free of a polyamide and the multilayered infusion container needed to have an oxygen scavenger layer sandwiched between the layers. However, this oxygen scavenger layer is not placed in direct contact with the aqueous solution of pemetrexed. That is, the oxygen scavenger layer is always placed in middle layers away from the innermost layer. The present inventors further discovered that multilayered flexible plastic infusion containers that have an oxygen scavenger layer sandwiched between polymeric layers and wherein the container is free of polyamide, when filled with aqueous solution of pemetrexed, could withstand autoclaving without any chemical or physical instability resulting from autoclaving at 121°C for 15 minutes. There was relatively little chemical degradation of pemetrexed and impurities of both hydrolytic nature and oxidative nature were controlled. Further there was no formation of particles of polyamide-11 cyclic dimer and/or polyamide-11 cyclic trimer, upon autoclaving. Other particles if any were within acceptable limits, not only immediately upon autoclaving but also on long term storage. There was no secondary packaging required before the step of autoclaving and therefore visual inspection could be directly done on the first container and then the inspected containers could be packed into secondary packaging. The intravenous infusion dosage form prepared by the process was advantageous in that when subjected to storage stability testing, the total impurity was not more than 2.0 % upon long term storage.

### SUMMARY OF THE INVENTION

The present invention provides an intravenous infusion dosage form comprising: an aqueous solution of pemetrexed or its pharmaceutically acceptable salt at a concentration ranging from 1.0 mg/ml to 20.0 mg/ml present in a multilayered flexible plastic infusion container, wherein the multilayered flexible plastic infusion container has an oxygen scavenger layer sandwiched between an outermost and an innermost layer of the container, the container being free of a polyamide and wherein the multilayered flexible plastic infusion container filled with the aqueous solution of pemetrexed is autoclavable.

The present invention may be summarized as follows:
i). An intravenous infusion dosage form comprising: an aqueous solution of pemetrexed or its pharmaceutically acceptable salt at a concentration ranging from 1.0 mg/ml to 20.0 mg/ml present in a multilayered flexible plastic infusion container, wherein the multilayered flexible plastic infusion container has an oxygen scavenger layer sandwiched between an outermost and an innermost layer of the container, the container being free of a polyamide and wherein the multilayered flexible plastic infusion container filled with the aqueous solution of pemetrexed is autoclavable.
ii). An intravenous infusion dosage form comprising: an aqueous solution of pemetrexed or its pharmaceutically acceptable salt at a concentration ranging from 1.0 mg/ml to 20.0 mg/ml present in a multilayered flexible plastic infusion container, wherein the multilayered flexible plastic infusion container has an oxygen scavenger layer sandwiched between an outermost and an innermost layer of the container, the container being free of a polyamide and wherein the multilayered flexible plastic infusion container filled with the aqueous solution of pemetrexed is autoclavable, wherein the oxygen scavenger layer of the intravenous infusion dosage form is made up of a polymer selected from a group consisting of ethylene vinyl alcohol copolymer and ethylene-vinyl acetate copolymer.
iii) An intravenous infusion dosage form comprising: an aqueous solution of pemetrexed or its pharmaceutically acceptable salt at a concentration ranging from 1.0 mg/ml to 15.0 mg/ml present in a multilayered flexible plastic infusion container, wherein the multilayered flexible plastic infusion container has an oxygen scavenger layer sandwiched between an outermost and an innermost layer of the container, the container being free of a polyamide wherein the oxygen scavenger layer is made up of a polymer selected from a group consisting of ethylene vinyl alcohol copolymer and ethylene-vinyl acetate copolymer and wherein the outermost layer of the intravenous infusion dosage form is made up of a polymer selected from a group consisting of polyethylene terephthalate, polypropylene terephthalate, polybutylene terephthalate and polyethylene naphthalate and/or wherein the innermost layer of the intravenous infusion dosage form is in direct contact with the aqueous solution of pemetrexed and is made up of a polymer selected from a group consisting of polyethylene and cycloolefin and wherein the multilayered flexible plastic infusion container filled with the aqueous solution of pemetrexed is autoclavable.

### DESCRIPTION OF THE FIGURES

FIGURE 1 provides the Raman spectrum of reference substance of polyamide-11 cyclic monomer.
FIGURE 2 provides the Raman spectrum of reference substance of polyamide-11 cyclic dimer.
FIGURE 3 provides the Raman spectrum of particles which were formed after autoclaving of the intravenous infusion dosage form of the aqueous solution of pemetrexed prepared as per Comparative Example II in which a multilayered flexible plastic infusion container made up of outer polyamide layer and having no oxygen scavenger layer was used.
FIGURE 4A provides the HPLC-MS chromatogram obtained when a solvent extract of polyamide resin sample of Sigma Aldrich containing a mixture of polyamide-11 cyclic monomer, polyamide-11 cyclic dimer and polyamide-11 cyclic trimer was injected. Three peaks were observed at retention time of 1.600, 2.019 and 2.438 minute.
FIGURE 4B provides the mass spectrum of the peak having a retention time of 2.438 minute (See Figure 4A). Its mass ion was 550.6 which corresponds to polyamide-11 cyclic trimer.
FIGURE 4C provides the mass spectrum of the peak having a retention time of 2.019 minute (See Figure 4A). Its mass ion was found to be 367.2 which corresponds to polyamide-11 cyclic dimer.
FIGURE 4D provides the mass spectrum of the peak having a retention time of 1.600 minute (Figure 4A). Its mass ion was found to be 184.6 which corresponds to polyamide-11 cyclic monomer.
FIGURE 5A provides the HPLC-MS chromatogram obtained when a solvent extract of the sub-visible particles as per Comparative Example III, was injected. Two peaks were observed at retention times of 2.438 and 2.019 minute.
FIGURE 5B provides the mass spectrum of the peak at retention time of 2.438 minute (See Figure 5A). Its mass ion was 550.7 which corresponds to polyamide-11 cyclic trimer.
FIGURE 5C provides the mass spectrum of the peak at retention time of 2.019 minute (See Figure 5A). Its mass ion was 367.2 which corresponds to polyamide-11 cyclic dimer.

### DETAILED DESCRIPTION OF THE INVENTION

The term "autoclavable' as used herein means that the multilayered flexible plastic infusion containers according to the present invention can withstand autoclaving without affecting the chemical and physical stability of aqueous solution of pemetrexed. By the term chemical stability, it is meant that the levels of impurities A, B and C are not more than 0.24 % by weight, respectively and the level of total impurity is not more than 2.0 % by weight of pemetrexed, when the autoclaved intravenous infusion dosage form according to the present invention is stored at room temperature for at least one year. By the term 'physical stability', it is meant that the aqueous solution of pemetrexed contained in the intravenous infusion dosage form according to the present invention when autoclaved and stored at room temperature for at least one year, the aqueous solution is found to be free of particles of polyamide-11 cyclic monomer and/or polyamide-11 cyclic dimer and/or polyamide-11 cyclic trimer. Other particles, if present, are found to be within the limits of particulate matter count specified for parenteral products by regulatory agencies, such as United States Pharmacopoeia Convention, Revision Bulletin 2011. United States Pharmacopoeia Convention specifies the limit of particulate matter count based on the volume of preparation in the container. For a container of nominal volume of 100 ml or less, the count of particles having size ≥10 µm should be not more than 6000 counts per container during the shelf life of the product and the count of particles having size ≥ 25 µm should be not more than 600 counts per container during the shelf life of the product. Also, for a container of nominal volume of more than 100 ml, the count of particles having size ≥10 µm should be not more than 25 particles per millilitre of the solution during the shelf life of the product and the count of particles having size ≥25 µm should be not more than 3 particles per millilitre of the container during the shelf life of the product. The count of particulate matter can be determined by techniques known in the art, such as microscopic particulate count or light obstruction particulate count.

By the term 'free of a polyamide' as used herein means that the container does not include polyamide material in any of its layers (which is also generally referred to as Nylon).

The term 'oxygen scavenger' as used herein means any material that possesses oxygen absorbing or oxygen scavenging property.

The term 'large volume' as used herein means that the volume of the aqueous solution in the containers is in the range of 50 ml to 1000 ml, such as for example 50, 60, 70, 80, 90, 100, 150, 200, 250, 300, 350, 400, 450, 500, 550, 600, 650, 700, 750, 800, 850, 900, 950 or 1000 ml.

The term 'multilayered flexible plastic infusion container' means a container having multiple layers of flexible films that are adhered, molded or sealed together by any means and have at least one layer made up of a plastic or polymeric material. The term multilayered means three or more layers. The term 'infusion container' means a container from which a large volume of the aqueous solution can be directly infused intravenously to the patient without further dilution.

The term 'innermost layer' refers to the layer of multilayered flexible plastic infusion container which is in direct contact with the aqueous solution of pemetrexed or its pharmaceutically acceptable salt.

The term 'ready-to-infuse' as used herein refers to the intravenous infusion dosage form which can be directly administered from the infusion container to the patients intravenously, without involving any intermediate steps of manipulation, dilution, reconstitution, dispensing, sterilization, transfer, handling or compounding before intravenous administration of the drug solution.

The term "sterile" as used in the context of the invention, means that the aqueous solution has been brought to a state of sterility and the solution complies with the sterility requirements of the standard Pharmacopoeias like United States Pharmacopoeia (USP) until the shelf life.

The term polyamide 11 polymer as used herein refers to a polymer formed by polymerization of 11 - aminoundecanoic acid, an amino acid having 11 carbon atoms.

The 11-aminoundecanoic acid and Polyamide 11 polymer are represented by Formula I and II respectively.

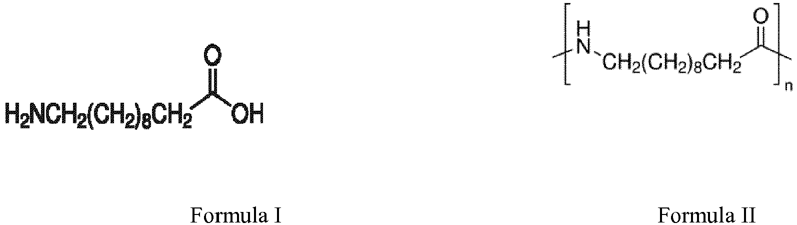

The polyamide 11 cyclic dimer and polyamide 11 cyclic trimers, as used herein in the specification are cyclic dimer and cyclic trimer of 11-aminoundecanoic acid, represented by Formula III and IV below respectively. Further structural details of the polyamide 11 cyclic dimer and polyamide 11 cyclic trimer are respectively described in journal references (1) Biopolymers, 2000 Oct 15; 54(5): 365-73 and (2) Macromolecules, 2001, 34: 837-843.

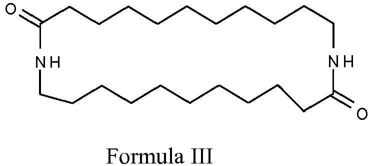

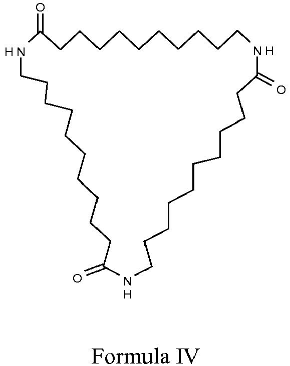

Impurity A, as used herein is a hydrolytic degradation impurity of pemetrexed and is chemically named as 4-[2-(2-Amino-4,7-dihydro-4-oxo-1H-pyrrollo [2,3-D] pyrimidine-5-yl) ethyl] benzoic acid. The chemical structure of impurity A is as follows:

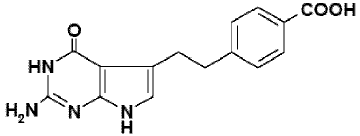

Impurity B, as used herein is an oxidative impurity of pemetrexed and is chemically named as (2S,2'S)-2,2'-[[(5R)-2,2'-diamino-4,4',6-trioxo-1,4,4',6,7,7'-hexahydro-1'H,5H-5,6'-bipryrolo[2,3-d] pyrimidine-5,5'-diyl]bis(ethylene benzene-4,1-diylcarbonylimino)] dipentanedioic acid. The chemical structure of impurity B is as follows:

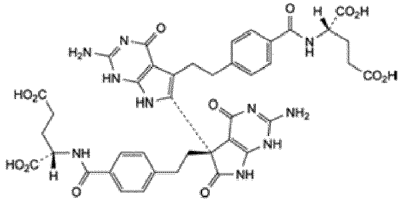

Impurity C, as used herein is an oxidative impurity of pemetrexed and is chemically named as (2S,2'S)-2,2'-[[(5S)-2,2'-diamino-4,4',6-trioxo-1,4,4',6,7,7'-hexahydro-1'H,5H-5,6'-bipyrrolo[2,3-d]pyrimidine-5,5'-diyl]bis(ethylenebenzene-4,1-diylcarbonylimino)]dipentanedioic acid. The chemical structure of impurity C is as follows:

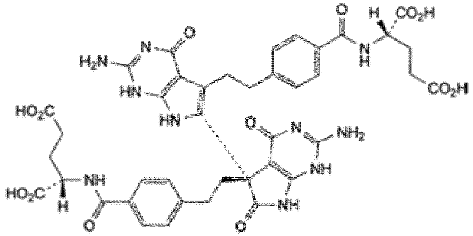

The Impurity F, as used herein is an oxidation impurity of pemetrexed and is chemically named as 4-{2-[(RS)-2-Amino-4,6 -dioxo-4, 5, 6, 7-tetrahydro-3H-pyrrolo[2,3-d]pyrimidin-5-yl]ethyl}benzoyl)-L-glutamic acid disodium. It is generally known as keto-pemetrexed and has the following chemical structure:

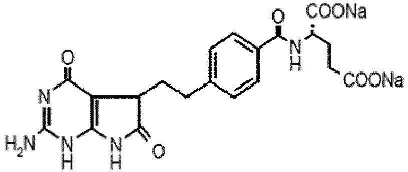

The levels of known, unknown and total impurities of pemetrexed present in the aqueous solution may be analyzed by any suitable means. Preferably, it may be analyzed by high performance liquid chromatography method. Any other suitable chromatographic technique may however be used.

The term 'total impurity' as used herein refers to summation of all known and unknown impurities of pemetrexed or its pharmaceutically acceptable salt. The total impurities are expressed as % by weight i.e. % of the labelled pemetrexed content present in the intravenous infusion dosage form of the present invention.

In specific embodiments, the intravenous infusion dosage form of the present invention comprises an aqueous solution of pemetrexed or its pharmaceutically acceptable salt filled in a multi-layered flexible plastic infusion container having more than two layers such as 3, 4, 5, 6, 7, 8, 9, 10, 11, 12, 13, 14 or 15 layers or more, wherein at least one layer of oxygen scavenger is sandwiched between the layers.

In preferred embodiments, the multilayered flexible plastic infusion container is made up of at least three layers comprising an oxygen scavenger layer which is sandwiched between an outermost and an innermost layer of the multi-layered flexible plastic container wherein the container is free of a polyamide and wherein the container filled with the aqueous solution of pemetrexed is autoclavable. In some embodiments, the multilayer flexible plastic infusion container can have other intermediate layers present in between the outermost layer and the oxygen scavenger layer and in between the oxygen scavenger layer and the innermost layer. In some embodiments, multi-layered flexible plastic infusion container have tie layer/s which sandwiches the layer having oxygen scavenger on both sides and helps the oxygen scavenger layer to bond with the other polymeric layers on either side.

The oxygen scavenger layer comprises one or more oxygen scavenger material which has oxygen absorbing or scavenging property. In one or more embodiments, the oxygen scavenger layer is made up of material selected from, but not limited to, ethylene vinyl alcohol copolymer, ethylene-vinyl acetate copolymer, metallocene polyethylenes, polymethylpentene; ethylene/vinyl aralkyl copolymer; atactic-1,2-polybutadiene, polyoctenamer, 1,4- polybutadiene; ethylene/vinyl cyclohexene copolymer; ethylene/methyl acrylate/ cyclohexenyl methyl acrylate terpolymer; homopolymer or a copolymer of cyclohexenylmethyl acrylate; ethylene/methyl acrylate/ cyclohexenylmethyl acrylate terpolymer, ethylene/ vinyl cyclohexene copolymer, ethylene/cyclohexenylmethyl acrylate copolymer and mixtures thereof. In some embodiment, the oxygen scavenger layer may have oxygen absorbing agents or catalysts as a part of the layer, for example transition metal salt of iron, nickel, copper, manganese, cobalt, rhodium, titanium, chromium, vanadium, ruthenium, and the like such as stearic acid cobalt, a neodecanoic acid cobalt; zeolites, silica based oxygen absorbers, iron based scavengers, charcoal etc. In preferred embodiments, the oxygen scavenger layer is made up of polymer selected from a group consisting of ethylene vinyl alcohol copolymer and ethylene-vinyl acetate copolymer. In a yet preferred embodiment, the oxygen scavenger layer is made up of ethylene vinyl alcohol copolymer. In specific embodiment, the oxygen scavenging layer has a thickness in the range of about 1 micron to about 80 micron, preferably about 5 micron to 20 micron, such as for example 5, 6, 7, 8, 9, 10, 11, 12, 13, 14, 15, 16, 17, 18, 19 or 20 micron. In one preferred embodiment, the oxygen scavenging layer has a thickness of 10 micron.

In one or more embodiments, the outermost layer of the multilayered flexible plastic infusion container according to the intravenous dosage form of the present invention is made up of a polymer selected from, but are not limited to, homopolymer or copolymer of polyalkylene terephthalates like polyethylene terephthylate (PET), polypropylene terephthalate, polybutylene terephthalate; polyalkylene naphthylates like polyethylene naphthalate, polypropylene naphthylate and the like. The outermost layer acts as a protective barrier layer. In some embodiments, the outermost layer may be made up of material like poly(vinyl alcohol), polyacrylonitrile, metallic foils, SiOx compounds, poly-styrenic base films, polyvinylidene dichloride. In preferred embodiments, the outermost layer of the flexible plastic infusion container is made up of a polymer selected from a group consisting of polyethylene terephthalate, polypropylene terephthalate, polybutylene terephthalate and polyethylene naphthalate. In one preferred embodiment, the outermost layer is made up of polyethylene terephthalate (PET). The outermost layer is also free of polyamide. In preferred embodiments, the outermost layer has a thickness in the range of about 5 micron to about 50 microns, preferably 5 to 20 microns such as for example 5, 6, 7, 8, 9, 10, 11, 12, 13, 14, 15, 16, 17, 18, 19 or 20 micron. In one preferred embodiment, the outermost layer has a thickness of 12 micron.

In one or more embodiments, the innermost layer of the multilayered flexible plastic infusion container which is in direct contact with the aqueous solution of pemetrexed is made up of polymeric materials selected from but not limited to polyalkylene polymers or copolymers like polyethylene polymer or copolymer; cycloolefin homopolymers or co-polymers like cycloolefin polymer; ethylene propylene block copolymer; ethylene/alpha-olefin copolymer; oxygen permeating polyolefins and the like. In an alternate embodiment, the innermost layer may be made up of polypropylene polymer or copolymer. The innermost layer is free of polyamide. Preferably, the innermost layer is selected from the group consisting of polyethylene layer and cycloolefin layer. In one preferred embodiment, the innermost layer is composed of polyethylene based polymer. Example of polyethylene polymers that are preferably used include, low density polyethylene polymer, linear low density polyethylene polymer, straight-chain low-density polyethylenes, super low density polyethylene, high density polyethylene polymer or a mixed composition thereof. In one preferred embodiment, the inner layer is composed of a low density polyethylene polymer which is linear or non-linear. In another preferred embodiment, the inner layer is composed of a high density polyethylene polymer. In one embodiment, a mixture of a linear low-density polyethylene and a high-density polyethylene is preferably used, in that the mixture has a property that supplements each other.

In an alternate embodiment, the innermost layer is composed of cyclo-olefin polymer or copolymer. The cyclic olefin polymer may be a cyclooolefin homopolymer (COP) or a cycloolefin copolymer (COC) or a mixture thereof. Cycloolefin homopolymers (cycloolefin polymers, or COP) are homopolymers comprising single type of cycloolefin monomers. Cycloolefins (cyclic olefins) are mono or polyunsaturated, mono or polycyclic ring systems such as cycloalkenes (like cyclopropene, cyclopentene, cyclobutene, cyclohexene), bicycloalkenes (like norbornene, dicyclopentadiene), tricycloalkenes, tetracycloalkenes (tetracyclododecene) and the like. The ring system can be monosubstituted or polysubstituted. Cycloolefin copolymers (COC) comprise cycloolefins and comonomers, wherein cycloolefins are copolymerized with one or more comonomers. Suitable comonomers are unsubstituted or substituted olefins, of 2 to 20 carbon atoms, preferably 2 to 6 carbon atoms, such as ethylene, butylene, etc. Any of these olefins may be used individually, or two or more types of olefins may be used in combination. In preferred embodiments, the innermost layer is made up of a polymer selected from ultra-low density polyethylene, low density polyethylene, linear low density polyethylene, medium density polyethylene, high density polyethylene or cycloolefin polymer and is free of a polyamide. In one preferred embodiment, the innermost layer of the flexible plastic infusion container that in direct contact with the aqueous solution is made up of a polymer selected from a group consisting of polyethylene and cycloolefin. The innermost layer has a thickness in the range of about 10 microns to about 220 microns, preferably 10 micron to about 50 microns, more preferably 15 to 30 microns such as for example 15, 16, 17, 18, 19, 20, 21, 22, 23, 24, 25, 26, 27, 28, 29 or 30 micron. In one specific embodiment, the outermost layer has a thickness of 20 micron.

In one or more embodiments, the multilayered flexible plastic infusion container according to the present invention has a total thickness in the range of 50 micron to 500 micron; preferably in the range of 100 micron to 450 micron, more preferably in the range of 200 to 350 micron such as for example 210, 215, 220, 225, 230, 235, 240, 245, 250, 255, 260, 265, 270, 275, 280, 285, 290, 295, 300, 305, 310, 315, 320, 325, 330, 335, 340 345 or 350 micron. In one or more embodiments, the multilayered flexible plastic infusion container according to the present invention has an oxygen transmission rate of less than 100 cc/ (m²·day·atm), preferably less than 50 cc/ (m²·day·atm), more preferably less than 20 cc/ (m²·day·atm), more preferably less than 10 cc/ (m²·day·atm) and most preferably less than 1 cc/ (m²·day·atm). The multi-layered flexible plastic containers available in the art that do not have any oxygen scavenger layer have an oxygen transmission rate ranging from 400 to 1500 cc/ (m²·day·atm).

In a preferred embodiment, the multilayered flexible plastic infusion container comprises at least three layers including an outermost layer of a polymer selected from polyethylene terephthalate, polypropylene terephthalate, polybutylene terephthalate or polyethylene naphthalate, a middle layer comprising an oxygen scavenger and an innermost layer selected from a polyethylene layer or a cycloolefin layer. In a yet preferred embodiment, the multilayered flexible plastic infusion container is made up of at least three layers comprising an outermost layer of a polymer selected from a group consisting of polyethylene terephthalate, polypropylene terephthalate, polybutylene terephthalate and polyethylene naphthalate, a middle oxygen scavenger layer comprising an oxygen scavenger selected from a group consisting of ethylene vinyl alcohol copolymer and ethylene-vinyl acetate copolymer and an innermost layer made up of a polymer selected from a group consisting of polyethylene and cycloolefin.

In a yet preferred embodiment, the multilayered flexible plastic infusion container is made up of at least ten layers including an outermost layer of a polymer selected from a group consisting of polyethylene terephthalate, polypropylene terephthalate, polybutylene terephthalate and polyethylene naphthalate, a middle oxygen scavenger layer comprising an oxygen scavenger selected from a group consisting of ethylene vinyl alcohol copolymer and ethylene-vinyl acetate copolymer and an innermost layer made up of a polymer selected from a group consisting of polyethylene and cycloolefin, wherein the multilayered flexible plastic infusion container is free of a polyamide and is autoclavable.

In a yet preferred embodiment, the multilayered flexible plastic infusion container is made up of thirteen layers including an outermost layer of polyethylene terephthalate, an oxygen scavenger layer made up of ethylene vinyl alcohol copolymer, adhesive tie layers on either side of oxygen scavenger layer, an innermost layer made up of high density polyethylene polymer and other inner layers present in between the outermost and oxygen scavenger layer and in between the oxygen scavenger and innermost layer. These inner layers are made up of linear low density polyethylene, high density polyethylene, cycloolefin and adhesive polymer. The multilayered flexible plastic infusion container is free of a polyamide.

In another embodiment, the multilayered flexible plastic infusion container comprises at least three layers including an outermost layer made up of polyethylene terephthalate, a middle layer made up of ethylene vinyl alcohol copolymer and an innermost layer made up of polyethylene polymer, wherein the multilayered flexible plastic infusion container is free of a polyamide and is autoclavable.

In a further preferred embodiment, the multilayered flexible plastic infusion container consists of thirteen layers including an outermost layer made up of polyethylene terephthalate, a middle layer made up of ethylene vinyl alcohol copolymer and an innermost layer made up of polyethylene polymer, wherein the multilayered flexible plastic infusion container is free of a polyamide and is autoclavable.

In a further preferred embodiment, the multilayered flexible plastic infusion container comprises at least three layers including an outermost layer made up of polyethylene terephthalate, a middle layer made up of ethylene vinyl alcohol copolymer and an innermost layer made up of cycloolefin polymer, wherein the multilayered flexible plastic infusion container is free of a polyamide and is autoclavable.

In one or more embodiments, the multilayered flexible plastic infusion container according to the present invention may further comprise additional layers present in between the outermost layer and middle oxygen scavenger layer and/or in between the middle oxygen scavenger layer and innermost layer. The one or more layers that may be present include layers made up of a material selected from a group consisting of polyethylene polymers and cyclo-olefin polymers as described above, such as for example high density polyethylene, low density polyethylene, linear low density polyethylene, cycloolefin polymer and cycloolefin copolymers.

In one or more embodiments, the multi-layered flexible plastic infusion container according to the present invention may further comprise adhesive layer/s, also referred to as lamination layer/s or tie layer/s. These adhesive layers may be present to affect proper binding of outer, intermediate and inner layers with each other. In one embodiment, the outermost layer is bound to an inner/middle layer comprising of oxygen scavenger by a dry lamination. In another embodiment, the outermost layer is adhered with an inner/middle layer comprising of oxygen scavenger by an adhesive resin layer. In one preferred embodiment, a tie layer is present on both sides of the oxygen scavenger layer, or in between the outermost layer and middle layers and/or middle layers and innermost layer. The adhesive layer may be made up of adhesive material selected from ethylene (meth) acrylic acid ester or copolymer, a modified EVA, epoxy resin composition, polyethylene-based resin adhesive which is selected from, but not limited to, linear low density polyethylene adhesive resin, high density polyethylene adhesive resin, straight chain low-density polyethylene adhesive resins. In another embodiment, the adhesive layer comprises a copolymer of α-olefin and a monomer of an unsaturated carboxylic acid or an anhydride of an unsaturated dicarboxylic acid. In one embodiment, the adhesive layer has a damp-proofing property. In another embodiment, the adhesive layer preferably contains a thermoplastic resin having adhesive properties. In one specific embodiment the tie layer sandwiches the layer comprising of oxygen scavenger. The tie layers may be made up of modified polyolefins blended with unmodified polyolefins or other suitable polymers. The modified polyolefins are typically polyethylene polymers or polyethylene copolymers. The modified polyethylenes can be ultra-low density polyethylene (ULDPE), low density polyethylene (LDPE), linear low density polyethylene (LLDPE), medium density polyethylene (MDPE), and high density polyethylenes (HDPE).

In one preferred embodiment, the present invention provides an intravenous infusion dosage form comprising an aqueous solution of pemetrexed or its pharmaceutically acceptable salt at a concentration ranging from 0.1 mg/ml to 20.0 mg/ml, preferably 2.0 to 15.0 mg/ml, filled in a multilayered flexible plastic infusion container, wherein the multilayered flexible plastic infusion container is free of a polyamide and is autoclavable, wherein the multilayered flexible plastic infusion container has an oxygen scavenger layer sandwiched between the multiple layers and wherein the outermost layer of the multilayered flexible plastic infusion container is made up of a polymer selected from a group consisting of homopolymer or copolymer of polyalkylene terephthalates and polyalkylene naphthylates like polyethylene terephthalate, polypropylene terephthalate, polybutylene terephthalate, polyethylene naphthalate or polypropylene naphthylate and the innermost layer is made up of a polymer selected from a group consisting of polyethylene and cycloolefin polymer.

In another embodiment, the present invention provides an intravenous infusion dosage form comprising an aqueous solution of pemetrexed or its pharmaceutically acceptable salt at a concentration ranging from 1.0 mg/ml to 20.0 mg/ml, preferably 2.0 to 15.0 mg/ml, filled in a multilayered flexible plastic infusion container, wherein the multilayered flexible plastic infusion container have an oxygen scavenger layer sandwiched between the multiple layers and wherein the multilayered flexible plastic infusion container is free of a polyamide and is autoclavable, wherein the multi-layered film of the multilayered flexible plastic infusion container has an oxygen transmission rate of less than 100 cc/ (m²·day·atm), preferably less than 50 cc/ (m²·day·atm), more preferably less than 1 cc/ (m²·day·atm). The multi-layered film has a total thickness in the range of 50 µm to 500 µm; preferably in the range of 100 µm to 450 µm, more preferably in the range of 200 to 350 µm. The outermost layer of the multilayered flexible plastic infusion container is made up of polyethylene terephthalate (PET) polymer and the innermost layer is made up of a polymer selected from a group consisting of polyethylene and cycloolefin polymer.

In a preferred embodiment, the present invention provides an intravenous infusion dosage form comprising an aqueous solution of pemetrexed or its pharmaceutically acceptable salt at a concentration ranging from 3.5 mg/ml to 13.0 mg/ml, such as for example 4.0, 4.5, 5.0, 5.5, 6, 6.5, 7.0, 7.5, 8.0, 8.5, 9.0, 9.5, 10.0, 10.5, 11.0, 11.5, 12.0, 12.5 or 13.0 mg/ml filled in a multilayered flexible plastic infusion container, wherein the multilayered flexible plastic infusion container comprises at least three layers including an outermost layer of a polymer selected from a group consisting of polyethylene terephthalate, polypropylene terephthalate, polybutylene terephthalate and polyethylene naphthalate, a middle layer made up of an oxygen scavenger selected from a group consisting of ethylene vinyl alcohol copolymer and ethylene-vinyl acetate copolymer, adhesive tie layers on either side of oxygen scavenger layer, an innermost layer made up of a polyethylene polymer or cycloolefin polymer, and optionally other inner layer present in between the outermost and oxygen scavenger layer and in between the oxygen scavenger and innermost layer. The inner layer/s can be made up of linear low density polyethylene, high density polyethylene, cycloolefin and adhesive polymer. The multilayered flexible plastic infusion container has oxygen transmission rate of less than 100 cc/ (m²·day·atm) and thickness of 50 µm to 500 µm, preferably 10 to 300 µm; further wherein the multilayered flexible plastic infusion container is free of a polyamide and is autoclavable.

In a preferred embodiment, the present invention provides an intravenous infusion dosage form comprising an aqueous solution of pemetrexed or its pharmaceutically acceptable salt, the aqueous solution consisting essentially of pemetrexed or its pharmaceutically acceptable salt at a concentration ranging from 2.0 mg/ml to 15.0 mg/ml, such as for example 2.0, 2.5, 3.0, 3.5, 4.0, 4.5, 5.0, 5.5, 6, 6.5, 7.0, 7.5, 8.0, 8.5, 9.0, 9.5, 10.0, 10.5, 11.0, 11.5, 12.0, 12.5, 13.0, 13.5, 14.0, 14.5 or 15.0 mg/ml, an osmotic agent, a pH adjusting agent and water for injection, wherein the aqueous solution has a pH in the range of 6.5 to 8.0 and is filled in a multilayered flexible plastic infusion container, wherein the multilayered flexible plastic infusion container comprises at least three layers including an outermost layer of a polymer selected from a group consisting of polyethylene terephthalate, polypropylene terephthalate, polybutylene terephthalate and polyethylene naphthalate, a middle layer made up of an oxygen scavenger selected from a group consisting of ethylene vinyl alcohol copolymer and ethylene-vinyl acetate copolymer, adhesive tie layers on either side of oxygen scavenger layer, an innermost layer made up of a polyethylene polymer or cycloolefin polymer, and optionally other inner layer present in between the outermost and oxygen scavenger layer and in between the oxygen scavenger and innermost layer. The inner layer can be made up of linear low density polyethylene, high density polyethylene, cycloolefin and adhesive polymer; wherein multilayered flexible plastic infusion container has oxygen transmission rate of less than 100 cc/ (m²·day·atm) and thickness of 50 to 500 µm; further wherein the multilayered flexible plastic infusion container is free of a polyamide and is autoclavable.

In yet another preferred embodiment, the present invention provides an intravenous infusion dosage form comprising an aqueous solution of pemetrexed or its pharmaceutically acceptable salt, the aqueous solution consisting essentially of pemetrexed or its pharmaceutically acceptable salt at a concentration ranging from 2.0 mg/ml to 15.0 mg/ml, such as for example 2.0, 2.5, 3.0, 3.5, 4.0, 4.5, 5.0, 5.5, 6, 6.5, 7.0, 7.5, 8.0, 8.5, 9.0, 9.5, 10.0, 10.5, 11.0, 11.5, 12.0, 12.5, 13.0, 13.5, 14.0, 14.5 or 15.0 mg/ml, an osmotic agent, a pH adjusting agent and water for injection, wherein the aqueous solution is free of added stabilizers such as antioxidants, amino acids, amines, complexing agents, cyclodextrins, chelating agents, co-solvents, alcohols, glycerine and propylene glycol; wherein the aqueous solution has a pH in the range of 7.0 to 7.5 and is filled in a multilayered flexible plastic infusion container, wherein the multilayered flexible plastic infusion container is made up of thirteen layers including an outermost layer of polyethylene terephthalate, a middle oxygen scavenger layer made up of ethylene vinyl alcohol copolymer, adhesive tie layers on either side of oxygen scavenger layer, an innermost layer made up of a polyethylene polymer and other inner layer/s present in between the outermost and oxygen scavenger layer and in between the oxygen scavenger and innermost layer, wherein the inner layer are made up of linear low density polyethylene, high density polyethylene, cycloolefin and adhesive polymer; wherein the multilayered flexible plastic infusion container has oxygen transmission rate of less than 100 cc/ (m²·day·atm) and thickness of 30 to 300 µm; further wherein the multilayered flexible plastic infusion container is free of a polyamide and is autoclavable.

In some embodiments, the present invention provides a set comprising two or more multilayered flexible plastic infusion containers comprising pemetrexed or its pharmaceutically acceptable, wherein the set caters to the cancer patients having a body surface area ranging from 1.3 to 2.4 m², wherein each infusion container is filled with an aqueous solution of pemetrexed comprising pemetrexed at a concentration ranging from 2.0 to 15.0 mg/ml, an osmotic agent, a pH adjusting agent to adjust the pH of the solution in the range of 7.0 to 8.0, wherein the solution is free of any antioxidants, amino acids, amines, complexing agents, cyclodextrins, chelating agents or co-solvents such as alcohols, glycerine or propylene glycol; wherein the multilayered flexible plastic infusion container comprises at least three layers including an outermost layer of a polymer selected from a group consisting of polyethylene terephthalate, polypropylene terephthalate, polybutylene terephthalate and polyethylene naphthalate, a middle layer made up of an oxygen scavenger selected from a group consisting of ethylene vinyl alcohol copolymer and ethylene-vinyl acetate copolymer, adhesive tie layers on either side of oxygen scavenger layer, an innermost layer made up of a polyethylene polymer or cycloolefin polymer, further wherein the multilayered flexible plastic infusion container is free of a polyamide and is autoclavable.

In one aspect, intravenous infusion dosage form of pemetrexed is useful in the treatment of non-squamous non-small cell lung cancer and mesothelioma. Preferably the dosage form is administered (alone or as a combined therapy with other agents) at a dose of 500 mg/m² intravenously, preferably as an infusion over a period of few minutes such as 5 to 30 minutes. Accordingly, each infusion container according to the present invention may provide the total dose in an intact, sterile container and the container include pemetrexed in amounts corresponding to the dose, which is based on the body surface area of the patient. In some embodiments, two or three containers together provide the total desired therapeutic dose. Based on this, the following embodiments are provided according to the present invention.

In one embodiment, there is provided a set according to the present invention comprising at least two multilayered flexible plastic infusion containers, each filled with same volume (for example 100ml) of aqueous solution of pemetrexed, wherein at least two containers comprises different concentration of pemetrexed in the range of 2.0 mg/ml to 15.0 mg/ml. For instance, there is provided a set of two multilayered flexible plastic infusion containers, one filled with 100 ml aqueous solution of pemetrexed having a concentration of 5.0 mg/ml while the other filled with 100 ml aqueous solution of pemetrexed having a concentration 10.0 mg/ml.

In another embodiment, there is provided a set according to the present invention comprising at least two multilayered flexible plastic infusion containers, each filled with same volume of aqueous solution of pemetrexed selected from 50 ml to 500 ml, and having same concentration of pemetrexed in the range of 2.0 to 15.0 mg/ml. For instance, there is provided a set of two multilayered flexible plastic infusion containers, each filled with 100 ml aqueous solution of pemetrexed, each having a concentration of 10.0 mg/ml.

In another embodiment, there is provided a set according to the present invention, comprising at least two multilayered flexible plastic infusion containers, each filled with aqueous solution of pemetrexed that have same concentration in the range of 2.0 to 15.0 mg/ml, but having different volumes selected from 50 ml, 60 ml, 70 ml, 80 ml, 90 ml, 100, 110, 120, 130, 140,150, 160, 170, 180, 190 or 200 ml. For instance, there is provided a set of two multilayered flexible plastic infusion containers, one filled with 100 ml aqueous solution of pemetrexed having a concentration of 10.0 mg/ml while the other filled with 50 ml aqueous solution of pemetrexed having a concentration 10.0 mg/ml.

In one preferred embodiment, there is provided a set according to the present invention comprising twenty multilayered flexible plastic infusion containers, each filled with 100 ml aqueous solution of pemetrexed, wherein the containers comprises different concentration of pemetrexed as follows, 3.5 mg/ml, 4.0 mg/ml, 4.5 mg/ml, 5.0 mg/ml, 5.5 mg/ml, 6.0 mg/ml, 6.5 mg/ml, 7.0 mg/ml, 7.5 mg/ml, 8.0 mg/ml, 8.5 mg/ml, 9.0 mg/ml, 9.5 mg/ml, 10.0 mg/ml, 10.5 mg/ml, 11.0 mg/ml, 11.5 mg/ml, 12.0 mg/ml, 12.5 mg/ml and 13.0 mg/ml.

In yet preferred embodiment, there is provided a set according to the present invention comprising thirteen multilayered flexible plastic infusion containers, each filled with 100 ml aqueous solution of pemetrexed, wherein the containers comprises different concentration of pemetrexed as follows, 3.5 mg/ml, 5.0 mg/ml, 6.0 mg/ml, 6.5 mg/ml, 7.0 mg/ml, 7.5 mg/ml, 8.0 mg/ml, 8.5 mg/ml, 9.0 mg/ml, 10.0 mg/ml, 11.0 mg/ml, 12.0 mg/ml and 13.0 mg/ml.

In another embodiment, there is provided a set according to the present invention comprising at least two multilayered flexible plastic infusion containers, each filled with same or different volume of aqueous solution of pemetrexed, wherein one of the container comprises aqueous solution of pemetrexed at a concentration ranging from about 2.0 mg/ml to 15.0 mg/ml and other container comprises aqueous solution of pemetrexed at a concentration ranging from about 0.1 to 1.9 mg/ml. For instance, there is provided a set of two multilayered flexible plastic infusion containers one filled with 100 ml aqueous solution of pemetrexed having a concentration of 13.0 mg/ml while the other filled with 100 ml aqueous solution of pemetrexed having a concentration 0.5 mg/ml. For instance, there is provided a set of two multilayered flexible plastic infusion containers one filled with 100 ml aqueous solution of pemetrexed having a concentration of 10.0 mg/ml while the other filled with 50 ml aqueous solution of pemetrexed having a concentration of 1.0 mg/ml. In this embodiment, the container having solution of lesser concentration in the range of 0.1 to 1.0 mg/ml can be used as a top up container along with the primary container having a concentration of 2.0 mg/ml to 15.0 mg/ml, to cater to desired dose of the anti-cancer drug for a particular indication.

According to one aspect, the intravenous infusion dosage form of pemetrexed according to the present invention is useful in the treatment of:
(i) Non-squamous, non-small cell lung cancer in combination with cisplatin, particularly for the initial treatment of patients with locally advanced or metastatic non-squamous, non-small cell lung cancer
(ii) Non-squamous, non-small cell lung cancer in combination with carboplatin and pembrolizumab, particularly for the initial treatment of patients with metastatic, non-squamous, non-small cell lung cancer
(iii) Non-squamous, non-small cell lung cancer as a single agent, particularly for the maintenance treatment of patients with locally advanced or metastatic non-squamous, non-small cell lung cancer whose disease has not progressed after four cycles of platinum-based first- line chemotherapy
(iv) Non-squamous, non-small cell lung cancer as a single agent, particularly for the treatment of patients with recurrent, metastatic non-squamous, non-small cell lung cancer after prior chemotherapy and/or
(v) Mesothelioma in combination with cisplatin, particularly for the initial treatment of patients with malignant pleural mesothelioma whose disease is unresectable or who are otherwise not candidates for curative surgery.

The intravenous infusion dosage form of pemetrexed according to the present invention is useful in the treatment of aforesaid indications, administered at a dose of 500 mg/m² as an intravenous infusion preferably over a period of 5 to 30 minutes, for example 10 minutes.

According to the present invention, the multilayered flexible plastic infusion container filled with the aqueous solution of pemetrexed, after autoclaving can be packaged in a secondary packaging having simple configuration during long term storage without any sophisticated or complicated configuration. Due to the unique configuration of the multilayered flexible plastic infusion container which oxygen scavenger layer sandwiched between the innermost and outmost layers, it allows use of a secondary packaging having simple configuration that does not have any special feature such as an oxygen scavenging or absorbing layer. That is, such secondary packaging can be a simple pouch or carton that does not have any sophisticated features such as oxygen barrier or scavenger. This offers lower cost as the secondary packaging does not contain any additives that can otherwise increase the cost. The secondary packaging may be in the form of an overwrap pouch or a bag or a film or a carton or other suitable package. The secondary packaging can be made up of an aluminum material such as for example aluminum pouch or film. According to the present invention there is no requirement to place a sachet of oxygen scavenger in the space between the multilayered flexible plastic infusion container and the secondary overwrap pouch.

The aqueous solution of pemetrexed filled in the multi-layered flexible plastic infusion container can include any pharmaceutically acceptable salt of pemetrexed such as those mentioned as sodium, disodium, potassium, lithium, calcium, magnesium, aluminum, zinc, ammonium, trimethylammonium, triethylammonium, monoethanolammonium, triethanol ammonium, tromethamine, pyridinium, substituted pyridinium salt and the like. Although, any suitable pharmaceutically acceptable salts of pemetrexed may be used, preferably, the pharmaceutically acceptable salt is pemetrexed disodium heptahydrate. The amount or concentration of pemetrexed or its pharmaceutically acceptable salts referred to in the present disclosure is expressed as amounts equivalent to pemetrexed free acid form. Pemetrexed or its pharmaceutically acceptable salt may be present in the aqueous solution in an amount ranging from about 0.1 mg/ml to about 20.0 mg/ml. In some preferred embodiments, pemetrexed or its pharmaceutically acceptable salt may be present in the aqueous solution in an amount ranging from about 2.0 mg/ml to 15.0 mg/ml, such as for example 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5 or 15.0 mg/ml, more preferably in an amount ranging from about 3.5 mg/ml to about 13.0 mg/ml. In some alternate embodiments, pemetrexed or its pharmaceutically acceptable salt may be present in the aqueous solution in an amount ranging from about 0.1 mg/ml to about 1.0 mg/ml, such as for example 0.2, 0.3, 0.4, 0.5, 0.6, 0.7, 0.8, 0.9 or 1.0 mg/ml.

The volume of the aqueous solution in each container is a large volume, meaning thereby that the volume ranges from about 50 ml to 1000 ml, preferably from about 80 ml to 500 ml, such as for example 80, 90, 100, 110, 120, 130, 140, 150, 160, 170, 180, 190, 200, 210, 220, 230, 240, 250, 300, 350, 400, 450 or 500 ml. In one preferred embodiment, the volume may range from about 100 ml to 240 ml. In some alternate embodiments, the volume may range from 250 ml to 500 ml.

In one or more embodiments, the dissolved oxygen content in the aqueous solution of pemetrexed in the intravenous infusion dosage form of the present invention is 2 parts per million (ppm) or less, i.e. 0 to 2 ppm, preferably 0 to 1.0 ppm, more preferably 0 to 0.5 ppm, such as for example 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20, 0.21, 0.22, 0.23,0.24, 0.25, 0.26, 0.27, 0.28, 0.29, 0.30, 0.31, 0.32, 0.33, 0.34, 0.35, 0.36, 0.37, 0.38, 0.39, 0.40, 0.41, 0.42, 0.43, 0.44, 0.45, 0.46, 0.47, 0.48 or 0.49 ppm. To achieve and maintain dissolved oxygen content in the range of 0 to 2 ppm, the aqueous solution is purged with an inert gas like nitrogen or argon. The dissolved oxygen content in the solution contained in the container may be determined by using dissolved oxygen meters such as marketed under the brand name Seven GO™, Seven Duo Go Pro™ (registered trademark -METTLER TOLEDO), or by using other methods known in the art such as Winkler-Azide titration method, method using diaphragm electrode (instrumental analysis) etc.

The aqueous solution can comprise of parenterally acceptable excipients such as, but not limited to, osmotic agents or tonicity adjusting agents, pH adjusting agents, surfactants or buffers. In one embodiment, an osmotic agent is used to adjust the tonicity of the solution and make the solution iso-osmolar to the parenteral/plasma fluids. The osmotic agent that may be used is selected from, but is not limited to sodium chloride, potassium chloride, mannitol, sorbitol, dextrose, sucrose and the like or mixtures thereof.

The aqueous solution of pemetrexed according to the present invention has a pH in the range of 6.0 to 11.0, preferably about 6.5 to 8.0, such as for example 6.6, 6.7, 6.8, 6.9, 7.0, 7.05, 7.10, 7.15, 7.20, 7.25, 7.30, 7.35, 7.40, 7.45, 7.50, 7.55, 7.60, 7.65, 7.70, 7.75, 7.80, 7.85, 7.90 or 7.95. The pH of the solution may be adjusted by use of a pH adjusting agent and if needed a buffer may be used to maintain the pH in the said range. The pH adjusting agent that may be used include, but are not limited to sodium hydroxide, potassium hydroxide, hydrochloric acid, sulfuric acid, acetic acid, sodium acetate, tartaric acid, and the like and mixtures thereof. In one preferred embodiment, the pH adjusting agent is sodium hydroxide and hydrochloric acid. The buffers or buffering agents that may be used to adjust and maintain the pH may be selected from a non-limiting group of pharmaceutically acceptable buffer systems such as citrate buffer, tartrate buffer, phosphate buffer, acetate buffer, lactate buffer, glycine buffer and the like or mixtures thereof. In one embodiment, the pH may be auto-adjusted by the ingredients present in the solution of the present invention.

The aqueous solution according to the present invention is free of antioxidants, complexing agents like cyclodextrins, chelating agents, and amino acids. It is important to note that although the aqueous solution of pemetrexed of the present invention is free of added stabilizers such as antioxidants, amino acids, amines, complexing agents such as cyclodextrins or co-solvents such as glycerine, propylene glycol, the dosage form is robust and chemically stable, even though subjected to autoclaving. It is physically and chemically stable and is also directly administrable in that the sterility is intact. This is a great advantage in the area of oncology parenteral drug delivery.

The aqueous solution of pemetrexed according to the present invention consists essentially of pemetrexed or its pharmaceutically acceptable salt, an osmotic agent, a pH adjusting agent and an aqueous vehicle like water for injection, wherein the aqueous solution has a pH in the range of 6.5 to 8.0. By the term "consisting essentially of' as used herein, it means that the aqueous solution of pemetrexed or its pharmaceutically acceptable salt according to the intravenous infusion dosage form of the present invention is free of added stabilizers such as antioxidants, amino acids, amines, complexing agents such as cyclodextrins, chelating agents or co-solvents such as alcohols, glycerine, propylene glycol and the like.

According to the present invention, the intravenous infusion dosage form of pemetrexed when subjected to autoclaving, the increase in the levels of total impurities in the aqueous solution of pemetrexed upon autoclaving is not more than 0.5 % by weight of pemetrexed and the solution does not show presence of polyamide-11 cyclic dimer and/or polyamide-11 cyclic trimer immediately upon autoclaving.

According to the present invention, when the intravenous infusion dosage form of pemetrexed is subjected to autoclaving, not more than 0.2 % by weight of impurity B or not more than 0.2 % by weight of Impurity C is present in the aqueous solution and the solution is free of particles of polyamide 11 -cyclic dimer or polyamide 11 -cyclic trimer, immediately upon autoclaving.

The intravenous infusion dosage form of pemetrexed according to the present invention is stable when stored at room temperature i.e. at 25°C/40%RH for at least one year or when stored at accelerated stability condition of 40°C/25% relative humidity for 6 months. The content of known oxidative impurities like impurity B and impurity C and the content of highest unknown impurity is not more than 0.24 % by weight of pemetrexed; preferably not more than 0.2 % by weight, more preferably not more than 0.15 % by weight of pemetrexed upon storage for the said period. The content of total impurities is not more than 2.0 % by weight of pemetrexed, preferably not more than 1.5 % by weight, more preferably not more than 1.0 % by weight of pemetrexed upon storage at room temperature i.e. at 25°C/40%RH for at least one year or when stored at accelerated stability condition of 40°C/25% relative humidity for 6 months. The six month accelerated storage stability data at 40°C/25% relative humidity corresponds to about two years storage stability at room temperature. The intravenous infusion dosage form of the present invention is stable over prolonged periods of at least one year for example up to one year, more preferably up to two years such that the content of hydrolytic impurity A is not more than 0.24 % by weight of pemetrexed and the content of oxidative impurity B, C and F is not more than 0.24 % by weight of pemetrexed and the content of highest unknown impurity is not more than 0.24 % by weight of pemetrexed and the content of total impurity is not more than 2.0 % by weight of pemetrexed.

The present invention further provides a process of preparing the intravenous infusion dosage form of pemetrexed, the process comprising steps of:
a) filling an aqueous solution comprising pemetrexed or its pharmaceutically acceptable salt at a concentration of 1.0 to 20.0 mg/ml in a multilayered flexible plastic infusion container, wherein the multilayered flexible plastic infusion container has an oxygen scavenger layer sandwiched between the outermost and innermost layers of the container and the container is free of a polyamide,
b) autoclaving the filled container of step (a)
   whereby upon autoclaving the aqueous solution of pemetrexed has total impurities not more than 0.5% by weight of pemetrexed and is free of polyamide 11-cyclic dimer or polyamide 11-cyclic trimer.

The present invention in one preferred embodiment provides a process of preparing a stable intravenous infusion dosage form of pemetrexed, the process comprising steps of:
a) filling an aqueous solution comprising pemetrexed or its pharmaceutically acceptable salt at a concentration of 2.0 to 15.0 mg/ml in a multilayered flexible plastic infusion container, the multilayers comprising at least three layers, an outermost layer, an oxygen scavenger layer and an innermost layer wherein the multilayered flexible plastic infusion container is free of a polyamide,
b) autoclaving the filled container of step (a) at a temperature in the range of 110°C to 125°C for a period of time in the range of 5 minutes to 60 minutes and sterilization pressure in the range of about 2.0 to 4.0 bar G,
   wherein the container is not packed or overwrapped by a secondary packaging during autoclaving,
c) packing the autoclaved multilayered flexible plastic infusion container in a secondary packaging.

In one specific embodiment, the present invention provides a process of preparing a stable intravenous infusion dosage form of pemetrexed, the process comprising the steps of:
a) preparing an aqueous solution consisting of pemetrexed or its pharmaceutically acceptable salt at a concentration of 2.0 to 15.0 mg/ml,
b) purging the solution with nitrogen,
c) filling the aqueous solution of (b) in a multilayered flexible plastic infusion container, the multilayers comprising at least three layers, an outermost layer, a middle oxygen scavenging layer and an innermost layer wherein the multilayered flexible plastic infusion container is free of a polyamide,
d) filling the head-space with inert gas and sealing the container;
e) autoclaving the filled container of step (d) by subjecting it to steam sterilization at temperature in the range of 120°C to 125°C for a period of time in the range of about 10 minutes to 25 minutes and a sterilization pressure of about 2.5 to 3.5 bar G, wherein the container is not packed or overwrapped by a secondary packaging during autoclaving
f) subjecting the autoclaved container of step (e) to visual inspection packing the inspected multilayered flexible plastic infusion container in a secondary packaging.

In one aspect, the present invention provides an intravenous infusion dosage form of pemetrexed prepared by a process comprising the steps of -
a) filling an aqueous solution comprising pemetrexed or its pharmaceutically acceptable salt at a concentration of 2.0 to 15.0 mg/ml in a multilayered flexible plastic infusion container, the multilayers comprising at least three layers, an outermost layer, an oxygen scavenger layer and an innermost layer wherein the multilayered flexible plastic infusion container is free of a polyamide,
b) autoclaving the filled container of step (a) at a temperature in the range of 110°C to 125°C for a period of time in the range of 5 minutes to 60 minutes and sterilization pressure in the range of about 2.0 to 3.5 bar G, wherein the container is not packed or overwrapped by a secondary packaging during autoclaving,
c) packing the autoclaved multilayered flexible plastic infusion container in a secondary packaging.

Embodiments are described herein as comprising certain features/elements. The disclosure also extends to separate embodiments consisting or consisting essentially of said features/elements.

Hereinafter, the invention is more specifically described by way of examples. The examples are not intended to limit the scope of the invention and are merely used as illustrations.

### COMPARATIVE EXAMPLE I

This comparative example demonstrates the problem associated with a pemetrexed solution in that even if the solution is purged with nitrogen and headspace filled with nitrogen, the total impurities increase significantly upon autoclaving.

**Table 1: Details of aqueous solution of pemetrexed**

| Ingredients | Concentration (% w/v) |
|---|---|
| Pemetrexed disodium heptahydrate eq. to Pemetrexed | 1.1 |
| Sodium Chloride | 0.9 |
| Sodium hydroxide and Hydrochloric acid | q.s. to adjust the pH at 7.2 |
| Water for Injection | q.s to 100 ml |

Sodium chloride was dissolved in water for injection. Nitrogen gas was purged into it to obtain dissolved oxygen level of less than 1 ppm. Pemetrexed disodium heptahydrate was then added to sodium chloride solution and the solution was stirred till pemetrexed sodium was dissolved. The pH of solution was adjusted to 7.2 using sodium hydroxide/ hydrochloric acid. The volume was made up with water for injection. The nitrogen gas purging was carried out continuously to maintain dissolved oxygen level of less than 1 ppm. The aqueous solution was then filtered through membrane filter of pore size 0.2 micron. 100 ml of the filtered aqueous solution was filled into each of the following multilayered flexible plastic containers:
(i) Multilayered flexible plastic container made up of multilayer polyolefin film having layers from outside to inside made up of - poly cyclohexanedimethyl cyclohexanedicarboxylate elastomer; functionalized ethylene alpha-olefin copolymer; ethylene alpha-olefin copolymer; styrene-ethylene-butylene-styrene block copolymer; and ethylene propylene copolymer, (referred as CPET-Tie-PE-Tie-EPC).
(ii) Multilayered flexible plastic container made up of an outer layer of polypropylene polymer with styrene-ethylene-butylene (SEB) block copolymer, middle and inner layer both made up of polypropylene based polyolefin polymer with styrene-ethylene butylene block copolymer.
(iii) Multilayered flexible plastic container made up of an inner layer of a cycloolefin polymer, a middle layer of linear low density polyethylene polymer and an outer layer of low density polyethylene polymer.

These infusion containers are free of oxygen scavenger in any of its layer.

The filled multi-layered plastic infusion containers (i), (ii), (iii) were sealed after replacing the headspace by nitrogen. The containers were not covered or wrapped and were as such subjected to autoclaving at 121°C for 15 minutes. The chemical stability was determined by measuring the % total impurity, % impurity B, C, F and % highest unknown impurity, before and after autoclaving by high performance liquid chromatography method, results whereof are presented below in Table 2:

**Table 2: Results of chemical analysis**

| Container type | Process Stage | Impurities | | | | |
|---|---|---|---|---|---|---|
| | | % Impurity B | % Impurity C | % Impurity F | % Highest Unknown Impurity | %Total Impurity |
| (i) | Before autoclaving | 0.029 | 0.040 | 0.020 | 0.029 | 0.207 |
| | After autoclaving | 0.287 | 0.381 | 0.111 | 0.247 | 1.177 (0.97 % increase) |
| (ii) | Before autoclaving | 0.029 | 0.037 | 0.018 | 0.036 | 0.193 |
| | After autoclaving | 0.295 | 0.365 | 0.113 | 0.223 | 1.154 (0.96 % increase) |
| (iii) | Before autoclaving | 0.032 | 0.043 | 0.033 | 0.031 | 0.214 |
| | After autoclaving | 0.240 | 0.299 | 0.105 | 0.216 | 1.019 (0.805 % increase) |

It was observed that in each of these containers, the level of impurities increased substantially upon autoclaving, for instance in container (i) the % impurity B, % impurity C and % highest unknown impurity increased by more than 8 fold from initial, whereby the amount of each impurities increased to more than 0.2 % upon autoclaving and the % impurity F increased by more than 5 folds. Also, there was a substantial increase in the % total impurities when the intravenous infusion dosage forms of pemetrexed according to comparative example I were subjected to autoclaving.

### COMPARATIVE EXAMPLE II

This comparative example demonstrated the discovery of the problem associated with the use of multilayered flexible plastic infusion container containing polyamide and not having an oxygen scavenger layer.

Aqueous solution of pemetrexed was prepared as per comparative example I and was filled into multilayered flexible plastic infusion container having the outermost layer made up of polyamide, middle layer made up of modified polyolefin and the innermost layer made up of polyethylene without having oxygen scavenger in any of its layer. The head space of the filled containers was replaced with nitrogen and then these containers were sealed. These sealed infusion containers were not covered or wrapped and were subjected as such to autoclaving at 121°C for 15 minutes. The chemical stability was determined by measuring the % total impurity, % impurity B, C, F and % highest unknown impurity, before and after autoclaving. The results of chemical analysis are presented below in Table 3.

**Table 3: Results of chemical analysis**

| Container type | Process Stage | Chemical Analysis | | | | |
|---|---|---|---|---|---|---|
| | | % Impurity B | % Impurity C | % Impurity F | % Highest Unknown Impurity | %Total Impurity |
| Multilayered plastic container having the outermost layer made up of Polyamide and having no oxygen scavenger layer | Before autoclaving | 0.034 | 0.046 | 0.023 | 0.032 | 0.206 |
| | After autoclaving | 0.255 | 0.331 | 0.093 | 0.231 | 1.069 (0.863% increase) |

It was observed that the level of impurities increased substantially upon autoclaving, for instance, the % impurity B, % impurity C and % highest unknown impurity increased by more than 7 fold from initial, whereby the content of impurities increased by more than 0.2 % upon autoclaving. Also, there was significant increase in the % total impurities when the intravenous infusion dosage form of comparative example II was subjected to autoclaving.

### COMPARATIVE EXAMPLE III

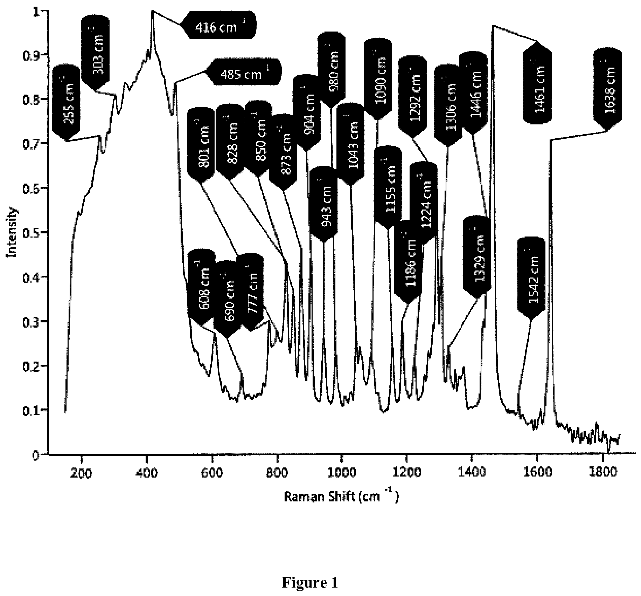

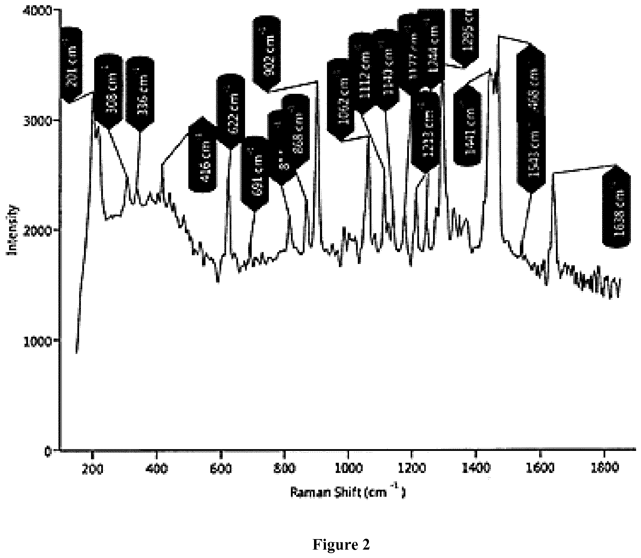

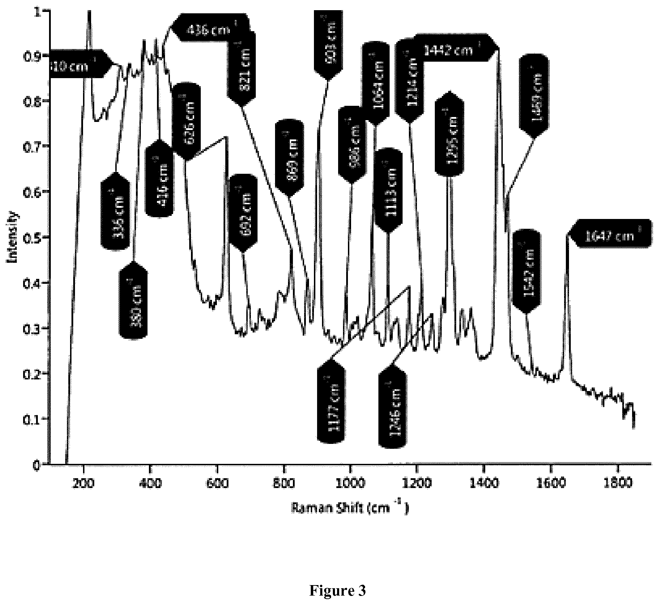

The physical observation of the aqueous solution of comparative example II indicated the presence of rod shaped sub-visible particles. These sub-visible particles were separated from the solution by filtration using 0.2µm Polyethersulfone filter and were subjected to characterization by Raman spectroscopy. The Raman spectrum of these particles was recorded by placing these filtered particles on Quartz plate of Raman G3 ID. The spectra are provided in Figure 3. The Raman spectrum of reference substances of polyamide 11 cyclic monomer and polyamide 11 cyclic dimer were recorded and are provided in Figure 1 and Figure 2, respectively. The prominent peaks observed at 700-1260 cm⁻¹ (functional group: C-C), 1410-1460 cm⁻¹ (Functional group: CH3 & CH2 deformations), and 1620-1690 cm⁻¹ (functional group: >C= O mixed with NH deformations) positions in Raman spectra of particles provided in Figure 3, matched to the peaks observed in same positions in Raman spectrum of polyamide reference substance of polyamide 11 cyclic monomer and polyamide 11 cyclic dimer provided in Figure 1 and Figure 2, respectively, indicating the presence of polyamide 11 cyclic monomer and polyamide 11 cyclic dimer in the particles.

The rod shaped sub-visible particles separated from the solution upon filtration as in comparative example II were further characterized by mass spectroscopy using LC-MS/MS technique. A triple quadrupole mass spectrometer AB-Sciex API 3200 with atmospheric pressure chemical ionization (APCI) (with positive molecule ionization) was used for the analysis. Scanning was performed with a mass range m/z from 100 to 1350 Dalton.

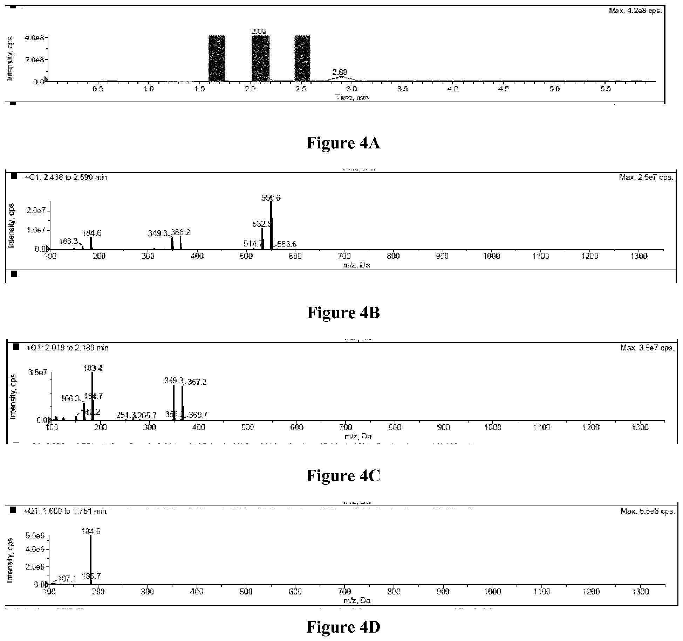

Reference substance - Preparation of polyamide-11 cyclic monomer, polyamide-11 cyclic dimer and polyamide-11 cyclic trimer reference standard and their mass spectroscopy: Polyamide resin was procured from Sigma Aldrich and was dissolved in a suitable solvent. The polyamide-11 cyclic monomer, polyamide-11 cyclic dimer and polyamide-11 cyclic trimer present in polyamide resin were separated from polyamide resin using preparatory HPLC. The separated polyamide-11 cyclic monomer, polyamide-11 cyclic dimer and polyamide-11 cyclic trimer were dissolved in methanol and mixed together to form a composite mixture. This methanolic solution containing composite mixture was injected into column of HPLC-Mass spectrometer and HPLC-MS chromatogram of mixture of reference substances of polyamide-11 cyclic monomer, polyamide-11 cyclic dimer and polyamide-11 cyclic trimer was recorded along with corresponding mass spectras. The HPLC-MS chromatogram showed a peak at retention time of 2.438 minute, 2.019 minute, and 1.600 minute as shown in Figure 4A. The mass spectrum at retention time 2.438 minute was of polyamide-11 cyclic trimer having a molecular ion mass of 550.6, as shown in figure 4B; the mass spectrum at retention time 2.019 minute was of polyamide-11 cyclic dimer having a molecular ion mass of 367.2, as shown in figure 4C; and the mass spectrum at retention time 1.600 minute was of polyamide-11 cyclic monomer having a molecular ion mass of 184.6, as shown in figure 4D.

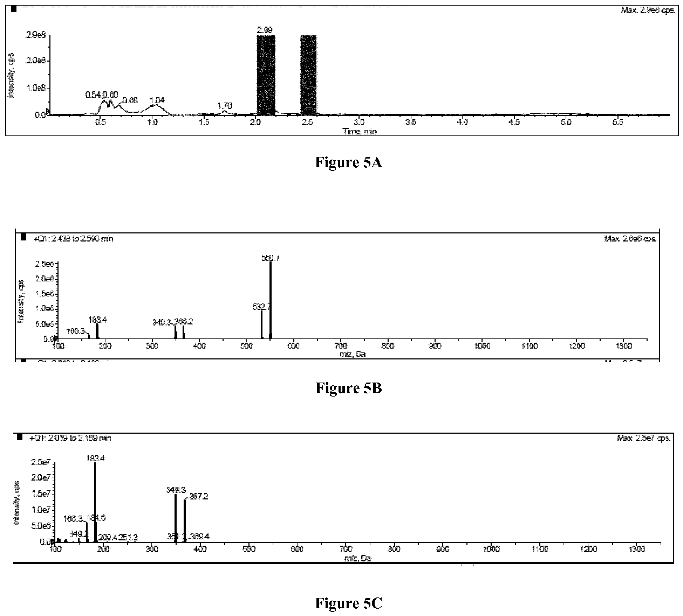

Mass spectroscopy of sub-visible particles - The rod shaped sub-visible particles separated from the solution upon filtration as in comparative example II were dissolved in methanol and the methanolic solution was injected into column of HPLC-mass spectrometer and chromatogram was recorded. The HPLC-MS chromatogram in figure 5A showed peaks at retention time 2.438 minute and 2.019 minute. The mass spectrum at retention time 2.438 min showed a molecular ion mass of 550.7 indicating presence of polyamide-11 cyclic trimer, as shown in Figure 5B and the mass spectrum at retention time 2.019 minute showed a molecular ion mass of 367.2 indicating presence of polyamide-11 cyclic dimer, as shown in Figure 5C.

The molecular ion mass (M+1)⁺ observed for the sub-visible particles as well as for the reference substances is provided below in Table 4.

**Table 4: Molecular ion mass of sub-visible particles and molecular ion mass of solvent extract from Polyamide resin sample procured from Sigma Aldrich.**

| Chemical name of the substance in solvent extract | Observed mass for reference substances, m/z = (M+1) | Observed mass for sub-visible particles of comparative example II m/z = (M+1) |
|---|---|---|
| Polyamide-11 Cyclic dimer | 367.2 | 367.2 |
| Polyamide-11 Cyclic trimer | 550.6 | 550.7 |

The results indicated that the observed molecular ion mass (M+1)⁺ of 550.7 (M+1)⁺ and 367.2 (M+1)⁺ for the sub-visible particles matched with the molecular ion mass of polyamide-11 cyclic dimer and polyamide-11 cyclic trimer respectively, thus confirming that the particles were of polyamide-11 cyclic dimer and polyamide-11 cyclic trimer.

### EXAMPLE 1

This example illustrates an infusion dosage form according to the present invention. The dosage form is prepared by following the steps given below:
Step (a) A solution of pemetrexed as described in comparative example I was prepared and was filled into a multilayered flexible plastic infusion container comprising an outermost layer made up of Polyethylene terephthalate, a middle oxygen scavenger layer (made up of ethylene vinyl alcohol copolymer) and an innermost layer made up of high density polyethylene polymer. The container was free of a polyamide layer. The headspace of filled multilayered flexible plastic infusion container was filled with inert gas and then the container was sealed.
Step (b) the filled container of step (a) was not covered or wrapped with a secondary packaging but was as such subjected to autoclaving at 121°C for 15 minutes in an autoclave.

The % total impurity, the % of known impurities B, C, F and the % of highest unknown impurity was quantified by high performance liquid chromatography method before and after autoclaving. The results are provided below in Table 5.

**Table 5: Results of chemical analysis**

| Stage | % Impurity B | % Impurity C | % Impurity F | % Highest Unknown Impurity | %Total Impurity |
|---|---|---|---|---|---|
| Before Autoclaving | 0.031 | 0.045 | 0.020 | 0.031 | 0.208 |
| After Autoclaving | 0.059 | 0.086 | 0.044 | 0.077 | 0.401 (Marginal increase of only 0.193%) |

| | | | | | |
|---|---|---|---|---|---|
| ND- Not Detected; RH- Relative humidity | | | | | |

The results in Table 5 indicated that upon autoclaving, the content of known impurities for example impurity B, impurity C and impurity F, the content of highest unknown impurity as well as content of total impurities were significantly lower compared to the corresponding levels observed in the containers of comparative examples I and II. The % total impurity in aqueous solution upon autoclaving was not more than 0.5 %. The increase in level of % total impurity in aqueous solution upon autoclaving was 'not more than 0.5 % increase'. The level of total impurities in example 1 according to the present invention does not increase substantially upon autoclaving, (marginal increase of only 0.193%) while in comparative example I {containers (i), (ii) and (iii)}, the content of total impurities increased substantially with an increase of 0.97 %, 0.96 % and 0.805 % respectively. Also in case of comparative example II, the content of total impurities increased substantially with an increase of 0.863 % upon autoclaving. In working example 1 according to the present invention, the increase in levels of other known impurities like impurity B, C or highest unknown impurity was marginal upon autoclaving while in comparative examples, the level of these impurities increased substantially. For instance, the content of impurity B in working example 1 according to the present invention was only 0.059 % by weight upon autoclaving, while in all comparative examples, the content of impurity B crossed 0.2 % by weight upon autoclaving.

This demonstrates the discovery of the inventors that if the first container has an oxygen scavenger middle layer it is not necessary to have the secondary packaging as in WO2016/129000 for the purpose of subjecting the infusion dosage form to autoclaving.
Step (c) The autoclaved containers obtained from step (b) were subjected to visual inspection.
Step (d) The inspected containers were packed in an aluminum pouch.

The stability of the packaged containers were tested at two different storage conditions, i.e. at 25°C/40% relative humidity (room temperature) and at 40°C/25% relative humidity (accelerated storage condition). The results of the analysis of impurities are given in Table 6.

**Table 6: Results of chemical analysis**

| Stage | % Impurity B | % Impurity C | % Impurity F | % Highest Unknown Impurity | %Total Impurity |
|---|---|---|---|---|---|
| Initial (Before Autoclaving) without secondary packaging | 0.031 | 0.045 | 0.020 | 0.031 | 0.208 |
| Initial (After Autoclaving) without secondary packaging | 0.059 | 0.086 | 0.044 | 0.077 | 0.401 |
| At 6Month storage at 25°C/40%RH with secondary packaging | 0.073 | 0.079 | 0.039 | 0.085 | 0.447 |
| At 12 Month storage at 25°C/40%RH with secondary packaging | 0.069 | 0.078 | 0.044 | 0.07 | 0.403 |
| At 3Month storage at 40°C/25%RH with secondary packaging | 0.067 | 0.074 | 0.038 | 0.067 | 0.403 |
| At 6 Month storage at 40°C/25%RH with secondary packaging | 0.077 | 0.082 | 0.043 | 0.093 | 0.488 |

| | | | | | |
|---|---|---|---|---|---|
| ND- Not Detected; RH- Relative humidity | | | | | |

Upon storage for six months at accelerated storage condition of 40°C/25% relative humidity and upon storage for twelve months at room temperature, i.e. at 25°C/40%RH, the content of known oxidative impurity like impurity B, impurity C or impurity F was not more than 0.1 % by weight of pemetrexed; the content of highest unknown impurity was not more than 0.1 % by weight of pemetrexed; and the content of total impurities not more than 1.0 % by weight of pemetrexed.

The examination of the aqueous solution was performed to check for presence of any visible or sub-visible particles. No visible particles were observed. Sub-visible particles were within specified limits. The sub-visible particles were separated from the aqueous solution by filtration using 0.2µm membrane filter. The filtered particles were analysed for their chemical nature. No particle of polyamide-11 cyclic monomer, polyamide-11 cyclic dimer and polyamide-11 cyclic trimer was found in the intravenous infusion dosage form according to example 1, either upon autoclaving or upon storage.

## Claims

1. An intravenous infusion dosage form comprising: an aqueous solution of pemetrexed or its pharmaceutically acceptable salt at a concentration ranging from 1.0 mg/ml to 20.0 mg/ml present in a multilayered flexible plastic infusion container, **characterised in that** the multilayered flexible plastic infusion container has an oxygen scavenger layer sandwiched between an outermost and an innermost layer of the container, the container being free of a polyamide and wherein the multilayered flexible plastic infusion container filled with the aqueous solution of pemetrexed is autoclavable.

2. The intravenous infusion dosage form according to claim 1 wherein the oxygen scavenger layer is made up of a polymer selected from ethylene vinyl alcohol copolymer or ethylene-vinyl acetate copolymer.

3. The intravenous infusion dosage form according to claim 1, wherein the outermost layer is made up of a polymer selected from polyethylene terephthalate, polypropylene terephthalate, polybutylene terephthalate or polyethylene naphthalate.

4. The intravenous infusion dosage form according to claim 1, wherein the innermost layer is in direct contact with the aqueous solution of pemetrexed and is made up of a polymer selected from polyethylene or cycloolefin.

5. The intravenous infusion dosage form according to claim 1, wherein not more than 0.20 % by weight of impurity B or not more than 0.20 % by weight of Impurity C is present in the aqueous solution and the solution is free of particles of polyamide 11-cyclic dimer or polyamide 11 -cyclic trimer, immediately upon autoclaving.

## Patentansprüche

1. Intravenöse Infusionsdarreichungsform umfassend: eine wässrige Lösung von Pemetrexed oder seinem pharmazeutisch akzeptablen Salz mit einer Konzentration, die von 1,0 mg/ml bis 20,0 mg/ml reicht, vorliegend in einem mehrschichtigen, flexiblen Kunststoff-Infusionsbehälter, **dadurch gekennzeichnet, dass** der mehrschichtige, flexible Kunststoff-Infusionsbehälter eine Sauerstofffängerschicht zwischen einer äußersten und innersten Schicht des Behälters aufweist, wobei der Behälter frei von einem Polyamid ist und wobei der mehrschichtige, flexible Kunststoff-Infusionsbehälter, der mit der wässrigen Lösung von Pemetrexed gefüllt ist, autoklavierbar ist.

2. Intravenöse Infusionsdarreichungsform nach Anspruch 1, wobei die Sauerstofffängerschicht aus einem Polymer besteht, das aus Ethylenvinylalkohol-Copolymer und Ethylenvinylacetat-Copolymer ausgewählt ist.

3. Intravenöse Infusionsdarreichungsform nach Anspruch 1, wobei die äußerste Schicht aus einem Polymer besteht, das aus Polyethylenterephthalat, Polypropylenterephthalat, Polybutylenterephthalat oder Polyethylennaphthalat ausgewählt ist.

4. Intravenöse Infusionsdarreichungsform nach Anspruch 1, wobei die innerste Schicht in direktem Kontakt mit der wässrigen Lösung von Pemetrexed ist und aus einem Polymer besteht, das aus Polyethylen oder Cycloolefin ausgewählt ist.

5. Intravenöse Infusionsdarreichungsform nach Anspruch 1, wobei nicht mehr als 0,20 Gew.-% von Verunreinigung B oder nicht mehr als 0,20 Gew.-% von Verunreinigung C in der wässrigen Lösung vorliegen und die Lösung unmittelbar nach dem Autoklavieren frei von Partikeln von Polyamid 11-zyklischem Dimer oder Polyamid 11-zyklischem Trimer ist.

## Revendications

1. Forme de dosage de perfusion intraveineuse comprenant : une solution aqueuse de pémétrexed ou de son sel pharmaceutiquement acceptable à une concentration allant de 1,0 mg/ml à 20,0 mg/ml présente dans un récipient de perfusion en plastique flexible multicouche, **caractérisé en ce que** le récipient de perfusion en plastique flexible multicouche a une couche capteuse d'oxygène prise en sandwich entre une couche la plus externe et une couche la plus interne du récipient, le récipient étant exempt d'un polyamide et dans lequel le récipient de perfusion en plastique flexible multicouche rempli avec la solution aqueuse de pémétrexed est autoclavable.

2. Forme de dosage de perfusion intraveineuse selon la revendication 1, dans laquelle la couche capteuse d'oxygène est composée d'un polymère sélectionné parmi du copolymère éthylène-alcool vinylique ou du copolymère éthylène-acétate vinylique.

3. Forme de dosage de perfusion intraveineuse selon la revendication 1, dans laquelle la couche la plus externe est constituée d'un polymère choisi parmi le polyéthylène téréphtalate, le polypropylène téréphtalate, le polybutylène téréphtalate ou le polyéthylène naphtalate.

4. Forme de dosage de perfusion intraveineuse selon la revendication 1, dans laquelle la couche la plus interne est en contact direct avec la solution aqueuse de pémétrexed et est composée d'un polymère sélectionné parmi le polyéthylène ou la cyclooléfine.

5. Forme de dosage de perfusion intraveineuse selon la revendication 1, dans laquelle pas plus de 0,20 % en poids de l'impureté B ou pas plus de 0,20 % en poids de l'impureté C est présent dans la solution aqueuse, et la solution est exempte de particules de polyamide 11-cyclique dimère ou de polyamide 11-cyclique trimère, immédiatement après l'autoclavage.
